# EUROPEAN PATENT APPLICATION

(11) **EP 4 492 079 A1**
(43) Date of publication of application: **15.01.2025**
(21) Application number: 23184786.4
(22) Date of filing: 11.07.2023
(51) Int. Cl.: G01R 33/54, A61B 5/055

(54) **AUTOMATED CONFIGURATION OF MAGNETIC RESONANCE IMAGING SYSTEMS**

(71) Applicant: Koninklijke Philips N.V., 5656 AG Eindhoven (NL)
(72) Inventor: HAHN, Artur, Eindhoven (NL); KEUPP, Jochen, Eindhoven (NL)
(74) Representative: Philips Intellectual Property & Standards

(57) **Abstract**

Disclosed herein is a medical system (100, 300) comprising a memory (110) storing machine executable instructions (120), a large language model module (112, 112'). The large language model module is configured to output a generated pulse sequence settings (126) in response to receiving subject metadata (124)as input. The generated pulse sequence settings are configured to control a magnetic resonance imaging system (302) to acquire k-space data (320). The medical system further comprises a computational system (104). The execution of the machine executable instructions causes the computational system to: receive (200) the subject metadata and receive (202) the generated pulse sequence settings in response to inputting the subject metadata into the large language model module.

## Description

### FIELD OF THE INVENTION

The invention relates to magnetic resonance imaging, in particular to automated configuration of magnetic resonance imaging systems.

### BACKGROUND OF THE INVENTION

A large static magnetic field is used by Magnetic Resonance Imaging (MRI) system or scanner to align the nuclear spins of atoms as part of the procedure for producing images within the body of a patient. This large static magnetic field is referred to as the B0 field or the main magnetic field. By controlling gradient magnetic fields and radio-frequency pulses the nuclear spins can be manipulated to produce radio-frequency signals that can be sampled or measured as k-space data. The k-space data can then be reconstructed into a magnetic resonance image which images internal anatomical structures of the subject. The time dependent control of the radio-frequency signals and radio-frequency pulses, as well as the sampling of the k-space data are described in pulse sequence settings. Properly configuring the magnetic resonance imaging system requires extensive training and experience.

### SUMMARY OF THE INVENTION

The invention provides for a medical system, a computer program, and a method in the independent claims. Embodiments are given in the dependent claims.

In one aspect the invention provides for a medical system that comprises a memory storing machine-executable instructions and a large language model module. The large language model module is configured to output a generated pulse sequence settings in response to receiving subject metadata as input. The generated pulse sequence settings are pulse sequence settings or data which may be converted into pulse sequence settings. The generated pulse sequence settings are configured to control a magnetic resonance imaging system to acquire k-space data.

The medical system further comprises a computational system. Execution of the machine-executable instructions causes the computational system to receive the subject metadata. Receiving the subject metadata may encompass receiving it as input from a user or from receiving it via a technical means such as retrieving it from the storage or retrieving it via a network. Execution of the machine-executable instructions further causes the computational system to receive the generated pulse sequence settings in response to inputting the subject metadata into the large language model module.

In another aspect the invention provides for a method that comprises receiving subject metadata. The method further comprises receiving generated pulse sequence settings in response to inputting the subject metadata into a large language model module. The large language model module is configured to output a generated pulse sequence settings in response to receiving subject metadata as input. The generated pulse sequence settings are configured to control a magnetic resonance imaging system to acquire k-space data.

In another aspect the invention provides for a computer program that comprises machine-executable instructions and also comprises a large language model module. The large language model module is configured to output a generated pulse sequence setting in response to receiving subject metadata as input. The generated pulse sequence settings are configured to control a magnetic resonance imaging system to acquire k-space data. Execution of the machine-executable instructions causes the computational system to receive the subject metadata. Execution of the machine-executable instructions further causes the computational system to receive the generated pulse sequence settings in response to inputting the subject metadata into the large language model module.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 3.
Fig. 5 illustrates an example of a large language model module.
Fig. 6 illustrates a further example of a large language model module.
Fig. 7 shows a flow chart which illustrates a further method.

### DETAILED DESCRIPTION OF EMBODIMENTS

Like numbered elements in these figures are either equivalent elements or perform the same function. Elements which have been discussed previously will not necessarily be discussed in later figures if the function is equivalent.

Examples may be beneficial because it may provide for a means of automatically generating pulse sequence settings for controlling a magnetic resonance imaging system. This may for example enable the use of a magnetic resonance imaging system independent of the presence of a technician.

Large language models (LLMs), like the architecture behind chatGPT, have demonstrated great capabilities to recall detailed information while handling flexible natural language queries in diverse scenarios, being trained on general public knowledge from the internet. While generality is an asset of LLMs, they can be fine-tuned to specific domains and configured to output certain text format and structured information instead of free text. Examples may use LLMs to automate MRI protocol planning in a one-state or two-stage configuration. The two-stage configuration is described below.

One specialized LLM "agent" (the first large language model) takes in subject metadata. The subject metadata may include patient data (which might include age, weight, etc.) and the reason for the MRI (symptoms, referring physician conjectures, ICD-10 codes) and recommends suitable MRI sequences, grouped into sensible protocols (the set of magnetic resonance imaging protocols). Once clinical staff or a decision module has chosen a protocol, a second LLM agent (second large language model) pre-configures each sequence with settings suitable for the patient (the generated pulse sequence settings), based on their age, weight, possible implants or other relevant information (the subject metadata). When staff makes changes to recommended protocols or sequence settings, this can be used for reinforced learning with human feedback to continuously improve recommendations or fine-tune a model for the preferences of a specific site. The software solution can blend seamlessly into the user interface of the MRI scanner and it can be offered as a cloud service or with local model storage. This enables remote operated and autonomous MRI systems and directly aids clinical workflow, reducing workload for the staff.

A large language model module as used herein encompasses a software component or an executable code which comprises at least one large language model.

A large language model (LLM) as used herein encompasses a neural network architecture, typically built up of transformers (encoders and decoders) with self-attention layers and residual connections, that is a language model that has been trained using unlabeled text using self-supervised or semi-supervised learning. Typically, LLMs are trained using billions of words. LLMs may for example be trained in an autoregressive form where given a segment of text the model predicts the next word (token) or words (tokens). Another mode of training is where words or tokens within a sentence are missing and the LLM predicts the missing words or tokens. Both types of LLMs may be configured to be used in the so-called prompting paradigm where a text query or statement is input into the LLM and the LLM outputs a completion or statement. The LLMs described herein are configured to operate in the prompting paradigm. Example LLMs are GPT-3, GPT-4, BERT, LLaMA, and others. The LLM may be trained for specific tasks using reinforcement learning or by reinforcement learning from human feedback (RLHF). The output of a preexisting LLM may be adjusted using fine-tuning. In fine-tuning a new set of weights connecting the final layer of the language model may be trained with specific data. Typically, this is done by freezing the other weights (other than the final output layer) in the neural network so that only the final output and format is affected.

Pulse sequence settings as used herein encompass commands or data which can be converted into such commands for controlling a magnetic resonance imaging system to acquire k-space data.

In another example, execution of the machine-executable instructions further causes the computational system to acquire the k-space data by controlling the magnetic resonance imaging system with the generated pulse sequence settings. Execution of the machine-executable instructions further causes the computational system to reconstruct a magnetic resonance image from the k-space data.

In another example the medical system further comprises the magnetic resonance imaging system.

In another example the large language model module comprises a first large language model and a second large language model. The first large language model is configured to output a set of magnetic resonance imaging protocols in response to receiving the subject metadata as input. The second large language model is configured to output the generated pulse sequence settings in response to receiving a selected magnetic resonance imaging protocol as input. The generated pulse sequence settings are configured to control the magnetic resonance imaging system to acquire k-space data according to the selected magnetic resonance imaging protocol. Execution of the machine-executable instructions further causes the computational system to receive a set of magnetic resonance imaging protocols in response to inputting the subject metadata into the first large language model.

Execution of the machine-executable instructions further causes the computational system to provide the set of magnetic resonance imaging protocols using the medical system. In different examples this may take different forms. In some cases, the set of magnetic resonance imaging protocols may be provided to a further software or artificial intelligence component. In other examples providing the set of magnetic resonance imaging protocols may encompass making these available on a display or user interface for selection.

The first large language model may be implemented, for example using GPT-2, GPT-3, BERT, or LLaMA. The first large language model may be further trained by using fine-tuning as described above to provide the set of magnetic resonance imaging protocols. In response to training subject metadata, the output can be trained to output a set of text strings each describing a magnetic resonance imaging protocol. The set of text strings could for example be limited to a dictionary or limited set of text strings which may be combined together.

The second large language model may be implemented, for example using GPT-2, GPT-3, BERT, or LLaMA. The second large language model may be further trained by using fine-tuning as described above to provide the generated pulse sequence setting in response to receiving the selected magnetic resonance imaging protocol, i.e., the selected group of magnetic resonance imaging sequences. In some examples the second large language model also receives the original subject meta data also. In the fine-tuning process, the output can be limited such that it is in the proper form to be displayed as a timing diagram (which may be readily converted into commands used to control the magnetic resonance imaging system) or as commands used to directly control the magnetic resonance imaging system. For the fine-tuning process training data that comprises trial magnetic resonance imaging protocols (which are input during training) and ground truth pulse sequence settings. The training for example may be done using historical pairs of trial magnetic resonance imaging protocols and ground truth pulse sequence settings.

Execution of the machine-executable instructions further causes the computational system to receive the selected magnetic resonance imaging protocol in response to providing the set of magnetic resonance imaging protocols. The set of magnetic resonance imaging protocols comprises the selected magnetic resonance imaging protocol. As is mentioned above, there are a variety of ways in which the selected magnetic resonance imaging protocol can be chosen.

Execution of the machine-executable instructions further causes the computational system to receive the generated pulse sequence settings in response to inputting the selected magnetic resonance imaging protocol into the second large language model. This example may be beneficial because it may provide for a more transparent means of generating the pulse sequence settings using a large language model. Breaking the process into several steps may make it more controllable and less likely to generate incorrect results.

In another example the large language model module further comprises a decision module. The decision module is configured to output the selected magnetic resonance imaging protocol in response to receiving the set of magnetic resonance imaging protocols as input. The set of magnetic resonance imaging protocols is provided by inputting the set of magnetic resonance imaging protocols into the decision module. The selected magnetic resonance imaging protocol is received as an output of the decision module in response to receiving the set of magnetic resonance imaging protocols. In some cases, the decision module may also receive the subject metadata as an input and both of these may be used to determine the selected magnetic resonance imaging protocol.

In some examples the decision module may compare the set of magnetic resonance imaging protocols against a list or set of hardware constraints. For example, the decision module can incorporate hardware constraints, i.e., what kind of MR hardware is available at the site. Certain types of MR hardware may or may not be able to perform a particular magnetic resonance imaging protocol.

The medical system, may in some examples, also have sensors for providing sensor data, The sensors may include such devices as a microphones, a camera, and/or a three-dimensional cameras. The sensor data may provide such information as patient geometry or positioning. The patient geometry or positioning may aid in choosing the appropriate magnetic resonance imaging protocol. Such sensor data may also be provided to the second large language model to provide geometry settings in the generated pulse sequences.

In another example the decision module is implemented as a neural network. The decision module may be trained by using training data that comprises training sets of magnetic resonance imaging protocols and a ground truth selected magnetic resonance imaging protocol selected from each of the training sets of magnetic resonance imaging protocols. For example, deep learning may be used.

In another example the decision module is implemented as a recurrent neural network. The decision module may be trained by using training data that comprises training sets of magnetic resonance imaging protocols and a ground truth selected magnetic resonance imaging protocol selected from each of the training sets of magnetic resonance imaging protocols.

In another example the decision module is implemented as a convolutional neural network. The decision module may be trained by using training data that comprises training sets of magnetic resonance imaging protocols and a ground truth selected magnetic resonance imaging protocol.

In another example the decision module is implemented as a decision tree. Prior sets of magnetic resonance imaging protocols and a selected magnetic resonance imaging protocol from each of these prior sets may be used to configure or train the decision module.

In another example the decision module is implemented as an expert system. The expert system may be configured by adding logic which may be used to choose the selected magnetic resonance imaging protocol.

In another example the decision module is implemented as a graph database. The graph database may comprise nodes and links between the nodes. The nodes and links between the nodes may be used to encode sets of magnetic resonance imaging protocols with the subject metadata and possibly additional sensor data being used to choose the selected magnetic resonance imaging protocol.

The various methods above which are related to artificial intelligence may be trained by collecting the choices of magnetic resonance imaging protocols when physicians ordered or chose the particular pulse sequence settings and trained using supervised learning.

In another example the providing the set of the magnetic resonance imaging protocols comprise displaying them on a user interface. The selected magnetic resonance imaging protocols are received via selection of the protocol from the user interface.

In another example execution of the machine-executable instructions further causes the computational system to train the first large language model with the selected magnetic resonance imaging protocol using reinforcement learning. When the selected magnetic resonance imaging protocol is chosen by either the decision module or by a user using the user interface this choice can be used to train the first large language module.

In another example the members of the set of the magnetic resonance imaging protocols are described textually or described as being a sentence or a phrase.

In another example the members of the set of pulse sequence protocols are constructed from a library of pulse sequence components. The library of pulse sequence components may encompass complete pulse sequences or portions of pulse sequences which may be combined together. This example may be beneficial because it limits how the set of magnetic resonance imaging protocols can be constructed. This may for example prevent an incorrect magnetic resonance imaging protocol from being generated.

In another example execution of the machine-executable instructions further causes the computational system to train the first large language model with radiology case reports using supervised and/or reinforcement learning. The use of radiology case reports may be beneficial because these may not be generally available and large language models trained using the internet would not have the opportunity to be trained using radiology case reports. The radiology case reports may also represent preferences of a particular location and/or physician. Training the first large language model with these radiology case reports may have the benefit of customizing them to a particular physician or location.

In another example the large language model module comprises a single large language model. In this example there is only a single large language model and it is able to output the generated pulse sequence settings in response to receiving the subject metadata.

The single large language model may be implemented, for example using GPT-2, GPT-3, BERT, or LLaMA. The large language model may be further trained by using fine-tuning as described above to provide the set of magnetic resonance imaging protocols. In response to training subject metadata, the LLM can be trained to output directly the generated pulse sequence settings. In the fine-tuning process, the output can be limited such that it is in the proper form to be displayed as a timing diagram (which may be readily converted into commands used to control the magnetic resonance imaging system) or as commands used to directly control the magnetic resonance imaging system. For the fine-tuning process training data that comprises trial subject metadata and ground truth pulse sequence settings. The training for example may be done using historical pairs of trial subject metadata and ground truth pulse sequence settings.

In another example execution of the machine-executable instructions further causes the computational system to display at least a portion of the generated pulse sequence settings on a user interface. Execution of the machine-executable instructions further causes the computational system to receive modification commands from the user interface in response to displaying at least a portion of the generated pulse sequence settings on the user interface. Execution of the machine-executable instructions further causes the computational system to modify the generated pulse sequence settings using the modification commands. This example may be beneficial because it may provide for a means of editing the generated pulse sequence settings. This modification process may also be useful in further training of the large language model module. In the case where there is only a single large language model then this data may be used for training the single large language model. If it is an example where there are two large language models then this data may be used for training the second large language model.

In another example execution of the machine-executable instructions further causes the computational system to train the large language model with the modification commands using reinforcement learning.

In another example the subject metadata comprises subject height.

In another example the subject meta data comprises subject weight.

In another example the subject metadata comprises subject sex or gender.

In another example the subject metadata comprises subject medical records.

In another example the subject metadata comprises subject diagnosis code.

In another example the subject metadata comprises physician or healthcare provider-generated text.

In another example the subject metadata comprises subject-generated text.

In another example the subject metadata comprises (recorded and/or transcribed) spoken language by the subject expressing symptoms or health concerns

In another example the subject metadata comprises (recorded and/or transcribed) spoken language by the health care professional on the medical question to be answered by the imaging exam

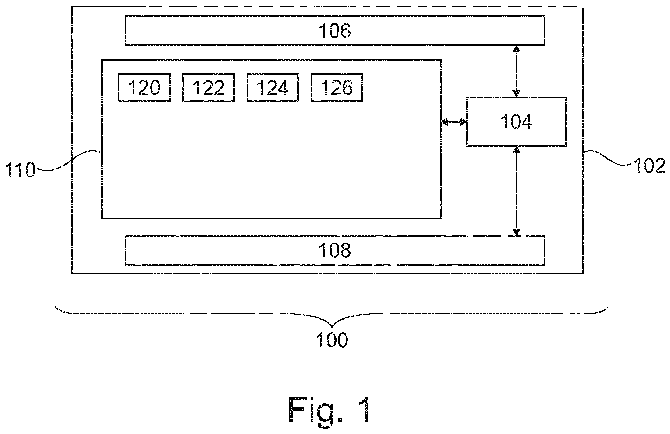

Fig. 1 illustrates an example of a medical system 100. This medical system 100 is shown as comprising a computer 102 that comprises a computational system 104. The computer 102 may represent one or more computers or computer systems at one or more locations. Likewise, the computational system 104 may represent one or more computational systems or cores that are located at one or more locations also. The computational system 104 is shown as being in communication with an optional hardware interface 106. If the medical system 100 comprises other components the hardware interface 106 may be used to communicate with and/or control those additional components. The computational system 104 is further shown as being in communication with an optional user interface 108 that may enable an operator or user to control and interact with the medical system 100.

The computational system 104 is further shown as comprising a memory 110. The memory 110 is intended to represent various types of volatile and non-volatile memory that is accessible to the computational system 104. In some examples the memory 110 may be a non-transitory storage medium.

The memory 110 is shown as containing machine-executable instructions 120. The machine-executable instructions 120 are instructions for the computational system 104 that enable it to perform various data analysis and control tasks. The memory 110 is further shown as containing a large language model module 122. The memory 110 is further shown as containing subject metadata 124 and generated pulse sequence settings 126. The generated pulse sequence settings 126 were received from the large language model module 122 in response to inputting the subject metadata 124 into it. The generated pulse sequence settings 126 are commands or data which may be converted into commands for controlling a magnetic resonance imaging system to acquire k-space data.

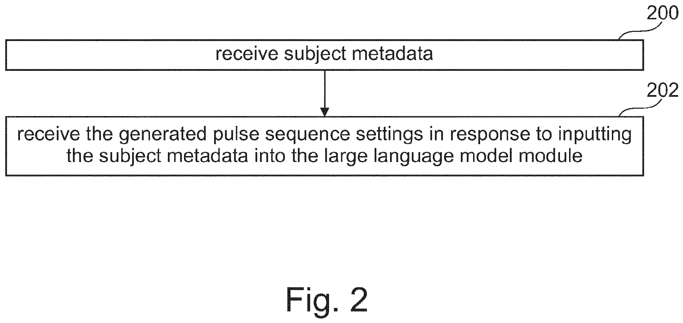

Fig. 2 shows a flowchart which illustrates a method of operating the medical system 100 of Fig. 1. In step 200 the subject metadata 124 is received. In step 202 the generated pulse sequence settings 126 are received by inputting the subject metadata 124 into the large language model module.

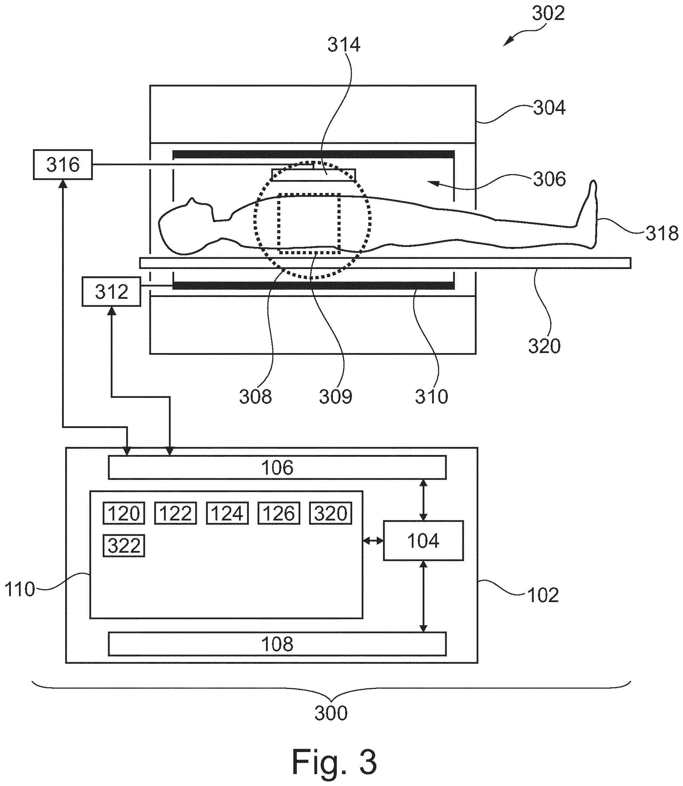

Fig. 3 illustrates a further example of a medical system 300. The medical system 300 depicted in Fig. 3 is similar to the medical system 100 in Fig. 1 except that it additionally comprises a magnetic resonance imaging system 302 that is controlled by the computational system 104.

The magnetic resonance imaging system 302 comprises a magnet 304. The magnet 304 is a superconducting cylindrical type magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data are acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for the acquisition of k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The memory 110 is further shown as containing k-space data 320 that was acquired by controlling the magnetic resonance imaging system 302 with the generated pulse sequence settings 126. The memory 110 is further shown as containing a magnetic resonance image 322 that was reconstructed from the k-space data 320.

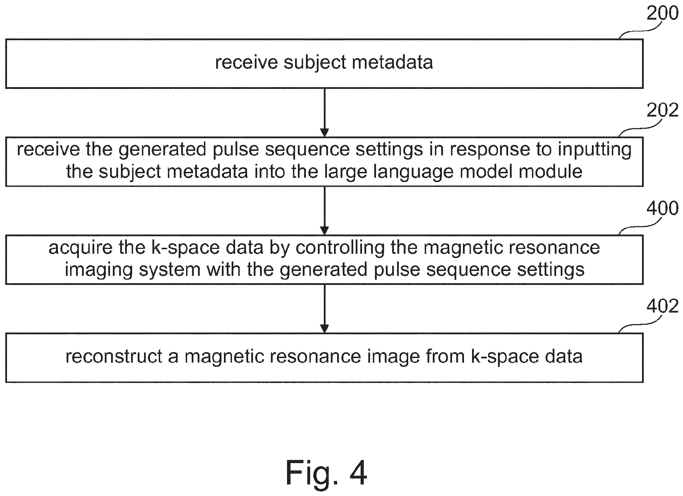

Fig. 4 shows a flowchart which illustrates a further method of operating the medical system 300 of Fig. 3. Steps 200 and 202 are performed as was illustrated in Fig. 2. In step 400 the k-space data 320 is acquired by controlling the magnetic resonance imaging system 302 with the generated pulse sequence settings 126. In step 402 the k-space data 320 is reconstructed into the magnetic resonance image 322.

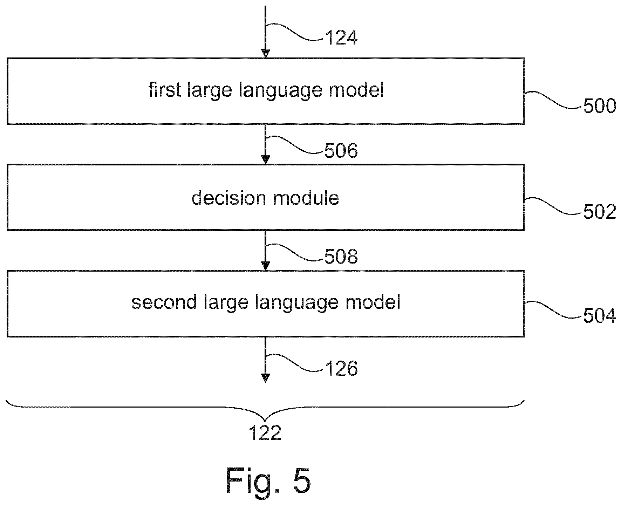

Fig. 5 illustrates an exemplary implementation of the large language model module 122. In this example, the large language model module 122 comprises a first large language model 500, a decision module 502, and a second large language model 504.

The first large language model 500 receives as input the subject metadata 124 and in response outputs a set of magnetic resonance imaging protocols 506. In examples, the set of magnetic resonance imaging protocols 506 may be a list or listing of different magnetic resonance imaging protocols in text or descriptive form.

The decision module 502 receives as input the set of magnetic resonance imaging protocols 506 and then outputs a selected magnetic resonance imaging protocol 508. The selected magnetic resonance imaging protocol 508 is one of the set of magnetic resonance imaging protocols 506. The decision module therefore is configured to make a choice from the set of magnetic resonance imaging protocols 506. In some examples the decision module 502 may also receive the subject metadata 124 as an input. In further examples the decision module receives sensor data. For example there may be cameras used to provide positional data descriptive of the subject or estimate the size and/or weight of the subject. The sensor data may be used to at least partially decide which magnetic resonance imaging protocol to select.

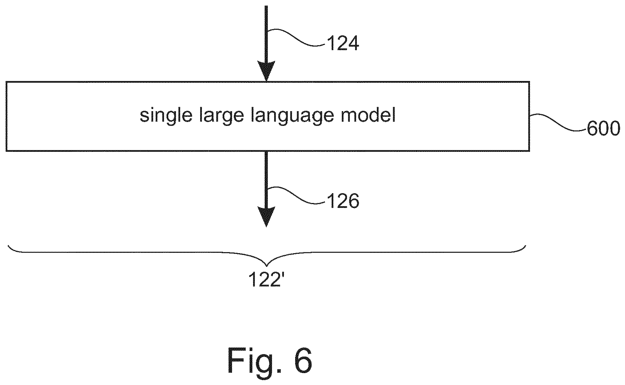

Fig. 6 illustrates an alternative implementation of the large language model module 122'. The large language model module 600 is configured to receive as input the subject metadata 124 and receive as output the generated pulse sequence settings 126.

Large language models (LLMs) as described above have revolutionized chat bots with milestones like ChatGPT. LLMs use encoder and decoder architectures with a large number of parameters (order billions-trillions) to learn large amounts of information in an unsupervised manner. For instance, LLMs can be trained with the entire public content of the internet to become a general-purpose chat bot.

LLMs functioning is based on predicting the next words of a conversation. Once pretrained (usually unsupervised) with more general data, LLMs can be fine-tuned with domain-specific data to teach them certain contexts, roles, rules, and types of responses. This is popularly done by incorporating supervised learning with training on exemplary dialogues or reinforcement learning from human feedback.

In examples, LLMs may be used for MRI protocol planning automation. This could intake a patient's data, including the reason for the MRI, i.e., symptoms, complaints, referring physician conjectures and wishes, and perhaps prior information from patient anamnesis/history. Suggesting an appropriate set of MRI scans for the patient and filling in the settings for each sequence could be handled by one or multiple LLMs fine-tuned for these specific tasks.

At a typical radiology site, a patient currently registers their information and reasons for the MRI (usually on a prescribing physician slip with a standardized ICD-10 code and/or some free text) upon arrival at the patient registration desk. Once the patient's turn has come, some MRI operating staff (radiologists, technicians) choose or put together a scan card for that patient with a set of suitable MRI scans based on their indications and personal data like implants, age or weight.

The resulting MRI examinations currently heavily rely on the experience of the local radiology staff. Locally modified vendor scan protocol settings vary throughout clinics and practices, and each team has individual preferences and experiences, leading to high imaging variability across departments and practices. An automation of MRI protocol planning would not only benefit clinical workflow and accelerate exam setup, freeing up valuable radiological staff time and increasing patient throughput, but it would also harmonize and slowly consolidate the different MRI sequences and settings used for different clinical questions, derived from symptoms and patient history.

Because of the need for highly trained humans to setup and conduct MRI examinations, the use is currently limited to hospitals and specialized radiology practices. Automation methods as proposed in this invention could enable MRI exams to be offered to retail customers for example in a shopping mall or on a cruise ship on a voluntary self-scan basis, without any specialized medical personnel present. Here, complaints, conjectures, symptoms or wishes for general check-ups expressed by the customer in non-expert language need to be translated into suitable MRI sequences for automatic operation which is not possible with the state-of-the-art.

Examples may comprise a software tool, incorporating large language models (LLMs) in the backend and an interface towards the scan planning software of an MRI machine at the frontend, including a user interface for the radiology staff to acknowledge or modify automatically proposed protocols. The LLM machine learning models are configured to return suitable MRI sequences and their detailed settings upon being queried with a specific patient case (with free-text, free speech and/or standardized medical codes), specifying, e.g., patient symptoms/complaints, patient anamnesis, referring physician conjectures, age, weight, sex, possible prior conditions, implants, pacemakers, and possibly other information relevant to choosing the optimal MRI protocol to answer the posed or possibly related clinical questions.

LLMs encompass a wider class of encoder and decoder architectures which can be trained in different ways and stages iteratively. Unsupervised learning can first be used to train an LLM with generally available knowledge, especially from the medical field (from textbooks, research publications, case reports, and other verified information sources). Supervised learning could then fine-tune the LLM for specific tasks and certain semantics and syntax of outputs, e.g., not outputting free text but instead MRI sequence settings files, as used natively by an MRI system (with possible adaptations to different vendor's systems and file formats). During field operation, the LLMs can further be fine-tuned continuously with user feedback, which can be screened and filtered before becoming model training data. Also, newly published medical information can constantly be incorporated through unsupervised, supervised, and reinforcement learning, updating the models incrementally.

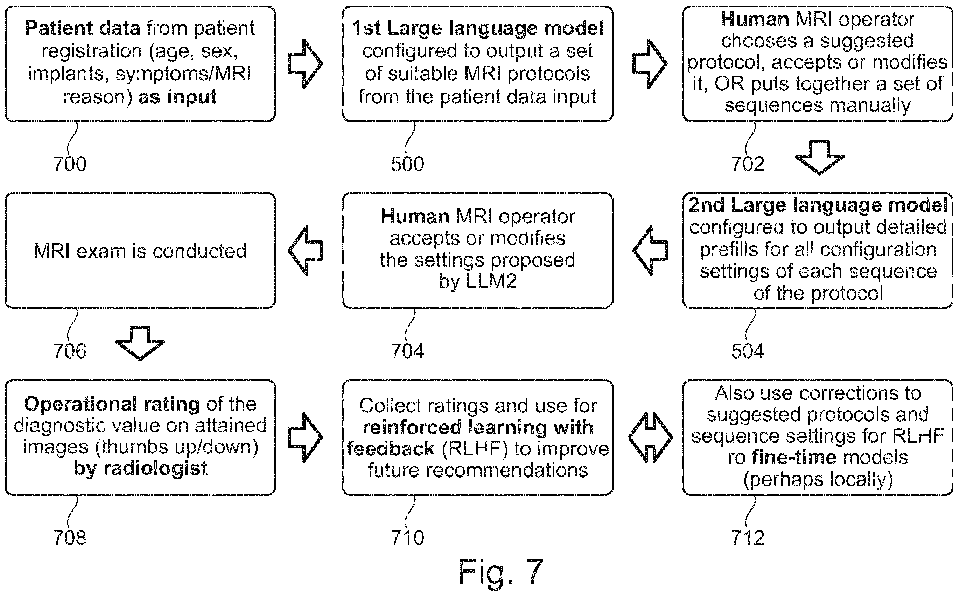

Fig. 7 shows a flowchart which illustrates a further example of the method. In step 700 patient data from a patient registration such as age, sex, implants, symptoms and MRI reason are provided as input. The patient data and other data are an example of subject metadata 124. In step 500 the first large language model 500 is configured to output a set of suitable MRI protocols for the set of magnetic resonance imaging protocols 506 in response to receiving the subject metadata 124 as input. In step 702 a human magnetic resonance imaging operator chooses a selected magnetic resonance imaging protocol 508. As an alternative the human MRI operator may reject all of these and put together a presence imaging protocol manually, which can be used for reinforcement learning.

In step 504 the second large language model is configured to output detailed prefills for all configuration settings for each sequence of the selected magnetic resonance imaging protocol 508. In step 704 the human magnetic resonance imaging operator accepts or modifies the settings proposed by the second large language model 504. In step 706 the magnetic resonance imaging exam is conducted and the k-space data 320 may be acquired and used to reconstruct the magnetic resonance image 322. In step 708 an optional rating of the diagnostic value on the magnetic resonance imaging is given a thumbs up or thumbs down by the radiologist. In step 710 the collected ratings are used for reinforced learning with human feedback or RLHF to improve future recommendations. In step 712 corrections to the suggested magnetic resonance imaging protocols and sequence settings for RLHF are used to fine-tune the models. This may be performed locally for a particular group or installation of magnetic resonance imaging systems to accommodate local preferences. The method illustrated in Fig. 7 is less automated than the other examples which were presented.

In some examples, implementations could blend into clinical workflow as described in Fig. 7. This should work on existing and older scanners. An adaptation for other vendors' MRI systems and software is also possible. The LLMs can be adapted (fine-tuned) to a certain system to output MRI settings in the required format. The hardware limitations of each system could be implemented in an iterative approach, details below.

At a sufficient training level of the LLMs for a defined range of medical conditions and imaging tasks, the system could operate without local human interference, either with remote supervision or in fully autonomous operation.

The interface of examples may integrate into the MRI protocol planning software. When opening a new scan card for a patient, the system will automatically retrieve the patient information associated with the individual (priorly entered during patient registration). The reasons for the MRI and patient particularities are sent as input to a first LLM:
1st LLM (first large language model 500): high-level scan card design assistant
- This LLM agent gives protocol suggestions with sensible sequences at a contrast/weighting/sequence type/name granularity
- Its recommendations are based on patient indications (symptoms, etc., provided in free-text, free speech or including medical expert information like codes) and human experience reflected in the training material of the LLM.

The MRI operating staff can choose a suggested protocol, modify it, or design their own. Once a set of MRI sequences has been selected, the second machine learning model incorporating LLM architecture receives the sequences as inputs:
2nd LLM (second large language model 504): detailed scan configuration assistant
- This LLM produces prefilled configurations for the chosen sequences (acquisition type, fat suppression, echo times, etc.)
- Prefills based on patient data like weight, age, implants, and possibly other data, including sensor data (e.g., cameras).
- This model can be fine-tuned to produce output in "MRI sequence settings language" (the generated pulse sequence settings 126), i.e., a file format containing all necessary information for the scan to be conducted, ideally in the vendor-native format.

In another example, the second LLM could be combined iteratively with other analytical and numerical tools, rule enforcing systems, or machine learning (like classifiers, decision trees, etc.), in order to iteratively refine the output to a certain query by switching between the creative, generative LLM output and a strict, rule-based supervisor system (like the current computational validation phases), which checks the conformity of the LLM recommendations with hardware capabilities, patient safety and regulations (somewhat reminiscent of iterations in a Generative Adversarial Network, GAN). If the supervising algorithm detects a conflict within recommended sequences or sequences settings, it could point out this conflict (and make suggestions) to the LLM and it could try to resolve or recommend alternatives, until a satisfactory solution is reached.

Modifications of the radiology staff can be incorporated to the algorithmic suggestions via reinforced learning with human feedback (RLHF), either to generally improve the performance of the models (feeding this feedback data into the globally maintained and distributed models), or also to fine-tune models to more local preferences, by updating the models with specific changes by the staff only for the locally used models.

In an example, on the MRI system and/or the diagnostic/viewing software, a 1-click feedback system could be offered to implement RLHF. During image viewing, radiologists could click a thumbs-up or thumbs-down icon in a corner of the image if it was helpful for diagnosis or especially useless and time wasting. This feedback can be collected and incorporated into training with special weights to have the recommender algorithms prefer high-scoring sequences and settings.

In yet another example, the LLMs could be augmented by different AI models and numerical tools to aid, monitor, and improve the recommendation algorithms.

The trained LLM models could be stored locally on computers at the MRI sites or hosted online by the maintaining vendor as a cloud service. User interactions with the system do not demand high bandwidth internet, since only relatively little text information is sent to and received from the proposed system to the user interface at the MRI machine. The communication and cloud-hosted models and services could be encrypted, only providing access to the specific site, e.g., through public/private key (end-to-end) encryption.

Examples may mimic parts of the cognitive, training and experience-based tasks that radiology staff are currently handling in full, but only after years of training and increasing experience; namely picking and configuring a (favorably fast) MRI protocol for a specific patient to acquire a sufficient amount of contrasts to answer related clinical questions. The proposed system gives its suggestions, building on a huge amount of knowledge which is constantly updated, aiding the MRI operator in designing a personalized sequence for the patient. The task of the operator shifts from manually configuring an exam to overseeing, approving, and tweaking suggested settings for each patient. This should relieve stress and time strain on staff, while reducing idle time of the scanner and patient wait times during scan setup.

Examples may facilitate remote and autonomous operation of an MRI, enabling scenarios where no highly trained radiology staff would have to locally operate and configure the scanner. Currently, radiology staff somewhere else could look at suggested scans for certain patient data and approve or modify the suggestions, configuring the scanner remotely. In future scenarios with highly refined models, the approval step may be skipped in some scenarios, like voluntary self-scanning in an MRI in a mall or on a cruise ship.

It is understood that one or more of the aforementioned examples of the invention may be combined as long as the combined examples are not mutually exclusive.

As will be appreciated by one skilled in the art, aspects of the present invention may be embodied as an apparatus, method or computer program product. Accordingly, aspects of the present invention may take the form of an entirely hardware example, an entirely software example (including firmware, resident software, micro-code, etc.) or an example combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some examples, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some examples computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments or examples of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

While the invention has been illustrated and described in detail in the drawings and foregoing description, such illustration and description are to be considered illustrative or exemplary and not restrictive; the invention is not limited to the disclosed embodiments or examples.

Other variations to the disclosed embodiments or examples can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: optional hardware interface
- 108: optional user interface
- 110: memory
- 120: machine executable instructions
- 122: large langue model module
- 122': large langue model module
- 124: subject metadata
- 126: generated pulse sequence settings
- 200: receive subject metadata
- 202: receive the generated pulse sequence settings in response to inputting the subject metadata into the large language model module
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio frequency coil
- 316: transceiver
- 318: subject
- 320: subject support
- 320: k-space data
- 322: magnetic resonance image
- 400: acquire the k-space data by controlling the magnetic resonance imaging system with the generated pulse sequence settings
- 402: reconstruct a magnetic resonance image from the k-space data
- 500: first large language model
- 502: decision module
- 504: second large language model
- 506: set of magnetic resonance imaging protocols
- 508: selected magnetic resonance imaging protocol
- 600: single large language model
- 700: patient data (subject metadata)
- 702: human MRI operator chooses the selected magnetic resonance imaging protocol
- 704: human MRI operator accepts or modifies the generated pulse sequence settings proposed by the second large language model
- 706: MRI examination is conducted
- 708: optional rating of the diagnostic value on attained images by radiologist
- 710: collect ratings and use for RLHF
- 712: use auto corrections to suggested protocols and sequence settings for RLHF to fine-tune models (perhaps locally)

## Claims

1. A medical system (100, 300) comprising:
- a memory (110) storing machine executable instructions (120), a large language model module (112, 112'), wherein the large language model module is configured to output generated pulse sequence settings (126) in response to receiving subject metadata (124) as input, wherein the generated pulse sequence settings are configured to control a magnetic resonance imaging system (302) to acquire k-space data (320);
- a computational system (104), wherein execution of the machine executable instructions causes the computational system to:
- receive (200) the subject metadata; and
- receive (202) the generated pulse sequence settings in response to inputting the subject metadata into the large language model module.

2. The medical system of claim 1, wherein execution of the machine executable instructions further causes the computational system to:
- acquire (400) the k-space data by controlling the magnetic resonance imaging system with the generated pulse sequence settings, and
- reconstruct (402) a magnetic resonance image (322) from the k-space data.

3. The medical system of claim 1 or 2, wherein the large language model module comprises a first large language model (500) and a second large language model (504), wherein the first large language model is configured to output a set of magnetic resonance imaging protocols (506) in response to receiving the subject metadata as input, wherein the second large language model is configured to output the generated pulse sequence settings in response to receiving a selected magnetic resonance imaging protocol as input, wherein the generated pulse sequence settings are configured to control the magnetic resonance imaging system to acquire k-space data according to the selected magnetic resonance imaging protocol, wherein execution of the machine executable instructions further causes the computational system to:
- receive a set of magnetic resonance imaging protocols in response to inputting the subject metadata into the first large language model;
- provide the set of magnetic resonance imaging protocols using the medical system;
- receive the selected magnetic resonance imaging protocol in response to providing the set of magnetic resonance imaging protocols, wherein the set of magnetic resonance imaging protocols comprises the selected magnetic resonance imaging protocol; and
- receive the generated pulse sequence settings in response to inputting the selected magnetic resonance imaging protocol into the second large language model.

4. The medical system of claim 1, 2, or 3, wherein the large language model module further comprises a decision module (502), wherein the decision module is configured to output the selected magnetic resonance imaging protocol in response to receiving the set of magnetic resonance imaging protocols as input, wherein the set of magnetic resonance imaging protocols are provided by inputting the set of magnetic resonance imaging protocols into the decision module, wherein the selected magnetic resonance imaging protocol is received as output of the decision module.

5. The medical system of claim 4, wherein the decision module is implemented as any one of the following: a neural network, a recurrent neural network, a convolutional neural network, a decision tree, an expert system, and a graph database.

6. The medical system of claim 3, wherein providing the set of magnetic resonance imaging protocols comprises displaying them on a user interface, and wherein the selected magnetic resonance imaging protocol is received via a selection of the protocol from the user interface.

7. The medical system of claim 4, 5, or 6, wherein the execution of the machine executable instructions further causes the computational system to train the first large language module with the selected magnetic resonance imaging protocol using reinforcement learning.

8. The medical system of any one of claims 3 through 6, wherein members of the set of magnetic resonance imaging protocols are described textually.

9. The medical system of any one of claims 3 through 8, wherein members of the set of pulse sequence protocols are constructed from a library of pulse sequence components.

10. The medical system of any one of claims 3 through 9, wherein execution of the machine executable instructions further causes the computational system to train the first large language module with radiology case reports using supervised and/or reinforcement learning.

11. The medical system of claim 1 or 2, wherein the large language model module comprises a single large language model (600).

12. The medical system of any one of the preceding claims, wherein execution of the machine executable instructions further causes the computational system to:
- display at least a portion of the generated pulse sequence settings on a user interface;
- receive modification commands from the user interface in response to displaying the at least a portion of the generated pulse sequence settings on the user interface;
- modify the generated pulse sequence settings using the modification commands.

13. The medical system of claim 12, wherein execution of the machine executable instructions further causes the computational system to train the large language model module with the modification commands using reinforcement learning.

14. A method comprising:
- receiving (200) subject metadata (124); and
- receiving (202) generated pulse sequence settings (126) in response to inputting the subject metadata into a large language model module (112, 112'), wherein the large language model module is configured to output the generated pulse sequence settings in response to receiving the subject metadata as input, wherein the generated pulse sequence settings are configured to control a magnetic resonance imaging system (302) to acquire k-space data (320).

15. A computer program comprising machine executable instructions (120) and a large language model module (112, 112'), wherein the large language model module is configured to output generated pulse sequence settings (126) in response to receiving subject metadata (124) as input, wherein the generated pulse sequence settings are configured to control a magnetic resonance imaging system (302) to acquire k-space data (320), wherein execution of the machine executable instructions causes the computational system to:
- receive (200) subject metadata; and
- receive (202) the generated pulse sequence settings in response to inputting the subject metadata into the large language model module.
